# EUROPÄISCHE PATENTSCHRIFT

(11) **EP 1 594 520 B1**
(45) Veröffentlichungstag und Bekanntmachung des Hinweises auf die Patenterteilung: **09.08.2006**
(21) Anmeldenummer: 04704552.1
(22) Anmeldetag: 23.01.2004
(51) Int. Cl.: A61K 38/04, A61P 19/02

(54) **DIE VERWENDUNG VON ANTAGONISTEN DES BRADYKININ-B2 REZEPTORS ZUR BEHANDLUNG VON OSTEOARTHROSE**
USE OF BRADYKININ-B2 RECEPTOR ANTAGONISTS FOR TREATING OSTEOARTHROSIS
UTILISATION D'ANTAGONISTES DU RECEPTEUR B2 DE LA BRADYKININE POUR TRAITER L'OSTEOARTHROSE

(30) Priorität: 07.02.2003 DE 10304994
(43) Veröffentlichungstag der Anmeldung: 16.11.2005
(73) Patentinhaber: Sanofi-Aventis Deutschland GmbH, 65929 Frankfurt am Main (DE)
(72) Erfinder: MICHAELIS, Martin, 60325 Frankfurt (DE); RUDOLPHI, Karl, 55116 Mainz (DE)
(86) Internationale Anmeldenummer: PCT/EP2004/000550
(87) Internationale Veröffentlichungsnummer: WO 2004/069266

(56) Entgegenhaltungen:
- EP-A- 0 370 453
- WO-A-03/063799

## Beschreibung

Die Erfindung betrifft die Verwendung von Peptiden mit Bradykinin-antagonistischer Wirkung zur Herstellung von Arzneimitteln zur Behandlung von degenerativen Gelenkserkrankungen.

In degenerativen Gelenkserkrankungen wie Osteoarthrose findet eine langsam fortschreitende Zerstörung des Gelenkes statt, die insbesondere durch den proteolytischen Abbau von Kollagen durch Kollagenasen bedingt ist. Kollagenasen gehören zur Superfamilie der Metalloproteinasen (MP) bzw. Matrix-Metalloproteinasen (MMPs). MMPs sind fähig, fibrilläres und nicht-fibrilläres Kollagen sowie Proteoglycane abzubauen, die sämtlich wichtige Bestandteile der Knorpelmatrix darstellen. MMP 3 ist am biologischen Abbau der extrazellulären Matrix beteiligt und wird in erhöhten Spiegeln bei Patienten mit Osteoarthrose gefunden, weshalb MMP 3 eine besondere Bedeutung beim Abbau der Gelenkmatrix in der Osteoarthrose zugesprochen wird (Manicourt et al. (1994), Arthritis and Rheumatism, 37:1774-83).

Bradykinin ist ein natürlich vorkommendes Nonapeptid, das einige pharmakologische Effekte aufweist, die zu Entzündungen und Schmerzen führen. Peptide mit Bradykinin-antagonistischer Wirkung werden bereits im Europäischen Patent EP 0 370 453 B1 beschrieben. Ferner ist bekannt, dass Peptide mit Bradykinin-antagonistischer Wirkung bei der Behandlung von Osteoarthritis oder rheumatoider Arthritis eingesetzt werden können (AU 638 350). Osteoarthritis und rheumatoide Arthritis sind Gelenkerkrankungen mit starken entzündlichen Phasen im Krankheitsverlauf. In Lerner et al. (Arthritis and Rheumatism (1987), 30, 530 - 540) wird berichtet, dass im Rahmen von rheumatoider Arthritis Bradykinin zwar die Knochenresorption verstärken kann, jedoch den Abbau der Knorpelmatrix selbst nicht stimuliert.

In dem Bestreben, wirksame Verbindungen zur Behandlung von degenerativen Gelenkserkrankungen zu finden, wurde nun gefunden, dass das erfindungsgemäß eingesetzte Peptid die Freisetzung von MMP's wie MMP-3 (sowie MMP-1 und MMP-13) hemmt. Dadurch kann der Matrixabbau wesentlich effektiver gehemmt werden, als nur durch die Inhibition von bereits freigesetzter oder im Gewebe gebildeten MMP's selbst.

Die Erfindung betrifft daher die Verwendung der Verbindung der Formel I

A-B-X-E-F-K-(D)-TIC-GM-M-F'-I (I)

zur Herstellung von Arzneimitteln zur Behandlung von degenerativen Gelenkserkrankungen, in welcher bedeuten:
- A: a₁) Wasserstoffatom, (C₁-C₈)-Alkyl, (C₁-C₈)-Alkanoyl, (C₁-C₈)-Alkoxycarbonyl oder (C₁-C₈)-Alkylsulfonyl, in welchen jeweils 1, 2 oder 3 Wasserstoffatome gegebenenfalls durch 1, 2 oder drei gleiche oder verschiedene Reste aus der Reihe Carboxy, Amino, (C₁-C₄)-Alkyl, (C₁-C₄)-Alkylamino, Hydroxy, (C₁-C₃)-Alkoxy, Halogen, Di-(C₁-C₄)-alkylamino, Carbamoyl, Sulfamoyl, (C₁-C₄)-Alkoxycarbonyl, (C₆-C₁₂)-Aryl und (C₆-C₁₂)-Aryl-(C₁-C₅)-alkyl ersetzt sind,
oder in welchen jeweils 1 Wasserstoffatom gegebenenfalls durch einen Rest aus der Reihe (C₃-C₈)-Cycloalkyl, (C₁-C₄)-Alkylsulfonyl, (C₁-C₄)-Alkylsulfinyl, (C₆-C₁₂)-Aryl-(C₁-C₄)-alkylsulfonyl, (C₆-C₁₂)-Aryl-(C₁-C₄)-alkylsulfinyl, (C₆-C₁₂)-Aryloxy, (C₃-C₉)-Heteroaryl und (C₃-C₉)-Heteroaryloxy
und 1 oder 2 Wasserstoffatome durch 1 oder 2 gleiche oder verschiedene Reste aus der Reihe Carboxy, Amino, (C₁-C₄)-Alkylamino, Hydroxy, (C₁-C₄)-Alkoxy, Halogen, Di-(C₁-C₄)-alkylamino, Carbamoyl, Sulfamoyl, (C₁-C₄)-Alkyloxycarbonyl, (C₆-C₁₂)-Aryl und (C₆-C₁₂)-Aryl-(C₁-C₅)-alkyl ersetzt sind,
a₂) (C₃-C₈)-Cycloalkyl, Carbamoyl,
   das gegebenenfalls am Stickstoff durch (C₁-C₆)-Alkyl oder (C₆-C₁₂)-Aryl substituiert sein kann,
   (C₆-C₁₂)-Aryl, (C₆-C₁₂)-Aryloyl, (C₆-C₁₂)-Arylsulfonyl oder (C₃-C₉)-Heteroaryl oder (C₃-C₉)-Heteroaryloyl, wobei in den unter a₁) und a₂) definierten Resten jeweils Heteroaryl, Aryloyl, Arylsulfonyl und Heteroaryloyl gegebenenfalls durch 1, 2, 3 oder 4 verschiedene Reste aus der Reihe Carboxy, Amino, Nitro, Hydroxy, Cyano, (C₁-C₄)-Alkylamino, (C₁-C₄)-Alkyl, (C₁-C₄)-Alkoxy, Halogen, Di-(C₁-C₄)-alkylamino, Carbamoyl, Sulfamoyl und (C₁-C₄)-Alkoxycarbonyl substituiert sind, oder
a₃) einen Rest der Formel II, 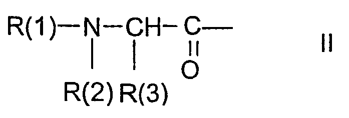 wobei
   R(1) wie A unter a₁) oder a₂) definiert ist,
   R(2) Wasserstoffatom oder Methyl bedeutet,
   R(3) Wasserstoffatom oder (C₁-C₆)-Alkyl, wobei Alkyl unsubstituiert oder durch Amino, substituiertes Amino, Hydroxy, Carbamoyl, Guanidino; substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert ist,
   wobei substituiertes Amino für einen Rest-NH-A- und substituiertes Guanidino für einen Rest -NH-C(NH)-NH-A stehen, in denen A wie unter a₁) oder a₂) definiert ist;
- B: Arg, Lys, Orn, 2,4-Diaminobutyroyl oder einen L-Homoargininrest,
wobei jeweils die Amino- oder die Guanidinogruppe der Seitenkette durch ein A wie unter a₁) oder a₂) beschrieben substituiert sein kann;
- X: eine Verbindung der Formel IIIa oder IIIb

G'-G'-Gly (IIIa)

G'-NH-(CH₂)ₙ-CO (IIIb),

worin G' unabhängige voneinander einem Rest der Formel IV 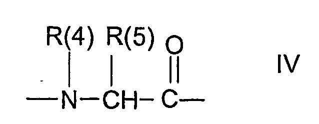 worin R(4) und R(5) zusammen mit den diesen tragenden Atomen ein heterocyclisches mono-, bi- oder tricyclisches Ringsystem mit 2 bis 15 C-Atomen bilden, und n 2 bis 8 ist;
- E: den Rest von Phenylalanin bedeutet,
das gegebenenfalls durch Halogen in der 2-, 3- oder 4-Ringposition substituiert ist, oder
Tyrosin, O-Methyltyrosin, 2-Thienylalanin, 2-Pyridylalanin oder Naphthylalanin bedeutet;
- F: unabhängig voneinander den Rest einer neutralen, sauren oder basischen, aliphatischen oder aromatischen Aminosäure bedeutet,
die in der Seitenkette substituiert sein kann,
oder eine kovalente Bindung bedeutet;
- (D)-Tic: den Rest der Formel V 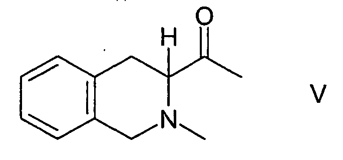
- G: G' bedeutet oder eine kovalente Bindung;
- F': den Rest einer basischen Aminosäure Arg oder Lys in der L- oder D-Form oder eine kovalente Bindung,
wobei die Guanidinogruppe oder Aminogruppe der Seitenkette durch A wie unter a₁) oder a₂) definiert substituiert sein kann,
oder einen Rest -NH-(CH₂)ₙ- mit n gleich 2-8,
oder eine kovalente Bindung bedeutet:,
- I: -OH, -NH₂ oder NHC₂H₅;
- K: den Rest -NH-(CH₂)ₓ-CO mit x gleich 1 bis 4 oder eine kovalente Bindung;
- M: wie F definiert,
sowie deren physiologisch verträgliche Salze

Ein weiterer Gegenstand der Erfindung ist auch die erfindungsgemäße Verwendung der Verbindung der Formel I, in welcher bedeuten:
- B: Arg, Orn oder Lys,
wobei die Guanidinogruppe oder die Aminogruppe der Seitenkette unsubstituiert ist oder durch (C₁-C₈)-Alkanoyl, (C₇-C₁₃)-Aryloyl, (C₃-C₉)-Heteroaryloyl, (C₁-C₈)-Alkylsulfonyl oder (C₆-C₁₂)-Arylsulfonyl substituiert sein kann, wobei die Aryl-, Heteroaryl-, Aryloyl, Arylsulfonyl- und Heteroaryloylreste wie oben unter a₂) beschrieben mit gegebenenfalls 1, 2, 3 oder 4 gleichen oder verschiedenen Resten substituiert sein können;
- E: Phenylalanin, 2-Chlorphenylalanin, 3-Chlorphenylalanin, 2-Fluorphenylalanin, 3-Fluorphenylalanin, 4-Fluorphenylalanin, Tyrosin, O-Methyltyrosin oder β-(2-Thienyl)-alanin;
- K: eine kovalente Bindung und
- M: eine kovalente Bindung ist.

Die Erfindung betrifft ferner die erfindungsgemäße Verwendung der Verbindung der Formel I, worin bedeuten:
- A: Wasserstoffatom, (D)- oder (L)-H-Arg, (D)- oder (L)-H-Lys oder (D)- oder (L)-H-Orn;
- B: Arg, Orn oder Lys,
wobei die Guanidinogruppe oder die Aminogruppe der Seitenkette durch Wasserstoffatom, (C₁-C₈)-Alkanoyl, (C₆-C₁₂)-Aryloyl, (C₃-C₉)-Heteroaryloyl, (C₁-C₈)-Alkylsulfonyl oder (C₆-C₁₂)-Arylsulfonyl substituiert sein kann,
wobei die Aryl, Heteroaryl-, Aryloyl, Arylsulfonyl- und Heteroaryloylreste gegebenenfalls mit 1, 2, 3 oder 4 gleichen oder verschiedenen Resten aus der Reihe Methyl, Methoxy und Halogen substituiert sein können;
- C: Pro-Pro-Gly, Hyp-Pro-Gly oder Pro-Hyp-Gly;
- E: Phe oder Thia;
- F: Ser, Hser, Lys, Leu, Val, Nle, ile oder Thr;
- K: eine kovalente Bindung
- M: eine kovalente Bindung
- G: den Rest eines heterocyclischen Ringsystems der Formel IV, ausgewählt aus den Resten der Heterocyclen Pyrrolidin (A), Piperidin (B), Tetrahydroisochinolin (C), cis- oder trans-Decahydroisochinolin (D), cis-endo-Octahydroindol (E), cis-exo-Octahydroindol (E), trans-Octahydroindol (E), cis-endo-, cis-exo-, trans-Octahydrocyclopentano[b]pyrrol (F), oder Hydroxyprolin (V);
- F': Arg;
- I: OH.

Die Erfindung betrifft auch die erfindungsgemäße Verwendung von einer Verbindung der Formel 1, dadurch gekennzeichnet, dass sie ausgewählt ist aus der Gruppe:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH und
H-(D)-Arg-Arg-Pro-Pro-Gly-Phe-Ser-(D)-Tic-Oic-Arg-OH.

Die Erfindung betrifft auch die erfindungsgemäße Verwendung von D-Arginyl-L-arginyl-L-prolyl-L-prolylglycyl-3-(2-thienyl)-L-alanyl-L-seryl-(3R)-1,2,3,4-tetrahydro-3-isochinolincarbonyl-(2S,3aS,7aS)-octahydro-1H-indol-2-carbonyl-L-arginin; das auch unter der Bezeichnung HOE140 bekannt ist.

Unter dem Begriff "(C₁-C₈)-Alkyl" werden Kohlenwasserstoffreste verstanden, deren Kohlenstoffkette geradkettig oder verzweigt ist und 1 bis 8 Kohlenstoffatome enthält, beispielsweise Methyl, Ethyl, Propyl, Iso-Propyl, Butyl, Iso-Butyl, tertiär-Butyl, Pentyl, IsoPentyl, Neopentyl, Hexyl, 2,3-Dimethylbutyl, Heptyl, Neohexyl oder Octyl.
Unter dem Begriff "Halogen" wird Fluor, Chlor, Brom oder Jod verstanden.
Unter dem Begriff "(C₃-C₈)-Cycloalkyl" werden Reste verstanden, die sich von 3- bis 8-gliedrige Monocyclen wie Cyclopropyl, Cyclobutyl, Cyclopentyl, Cyclohexyl, Cycloheptyl oder Cylooctyl herleiten.
Unter dem Begriff "-(C₆-C₁₂)-Aryl" werden aromatische Kohlenstoffreste verstanden mit 6 bis 14 Kohlenstoffatomen im Ring. -(C₆-C₁₂)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste..
Unter dem Begriff "(C₃-C₉)-Heteroaryl" werden Reste wie Acridinyl, Azetidinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chromanyl, Chromenyl, Cinnolinyl, Decahydrochinolinyl, 2H, 6H-1,5,2-Dithiazinyl, Dihydrofuran[2,3-b]-tetrahydrofuran, Furanyl, Furazanyl, Imidazolidinyl, Imidazolinyl, Imidazolyl, 1H-Indazolyl, indolinyl, indolizinyl, indolyl, 3H-Indolyi, isobenzofuranyl, Isochromanyl, Isoindazolyl, Isoindolinyl, isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-0xadiazolyl, Oxazolidinyl, Oxazolyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pyridooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Chinazolinyl, Chinolinyl, 4H-Chinolizinyl, Chinoxalinyl, Chinuclidinyl, Tetrahydrofuranyl, Tetrahydroisochinolinyl, Tetrahydrochinolinyl, 6H-1,2,5-Thiadazinyl,1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiophenyl, Triazinyl, 1,2,3-Triazolyl, 1,2,4-Triazolyl, 1,2,5-Triazolyl, 1,3,4-Triazolyl und Xanthenyl verstanden.
Bevorzugt sind Pyridyl; wie 2-Pyridyl, 3-Pyridyl oder 4-Pyridyl; Pyrrolyl; wie 2-Pyrrolyl und 3-Pyrrolyl; Furyl; wie 2-Furyl und 3-Furyl; Thiophenyl, Thienyl; wie 2-Thienyl und 3-Thienyl; imidazolyl, Pyrazolyl, Oxazolyl, Isoxazolyl, Thiazolyl, isothiazolyl, Tetrazolyl, Pyridazinyl, Pyrazinyl, Pyrimidinyl, Indolyl, isoindolyl, Benzofuranyl, Benzothiophenyl, 1,3-Benzodioxolyl, Indazolyl, Benzimidazolyl, Benzoxazolyl, Benzothiazolyl, Chinolinyl, Isochinolinyl, Chromanyl, Isochromanyl, Cinnolinyl, Chinazolinyl, Chinoxalinyl, Phthalazinyl, Pyridoimidazolyl, Pyridopyridinyl, Pyridopyrimidinyl, Purinyl und Pteridinyl.

Die erfindungsgemäß eingesetzten Peptide werden wie in EP 0 370 453 B1 beschrieben hergestellt.

Aufgrund der pharmakologischen Eigenschaften eignen sich die erfindungsgemäßen Verbindungen zur selektiven Prophylaxe und Therapie von degenerative Gelenkerkrankungen wie Osteoarthrose, Spondylose oder Knorpelschwund nach Gelenktrauma oder längerer Gelenksruhigstellung nach Meniskus- oder Patellaverletzungen oder Bänderrissen.
Unter dem Begriff "Osteoarthrose" wird eine Erkrankung verstanden, die vorwiegend bei einem Missverhältnis zwischen Beanspruchung und Belastbarkeit der einzelnen Gelenkanteile und Gelenkgewebe entsteht, die mit einer zunehmenden Knorpeldestruktion verbunden ist und vorwiegend nicht entzündlich ist. Im Vordergrund der Pathologie stehen Schädigungen des Gelenkknorpels wie Auffaserung, Demarkierung oder Hyalinisierung, gefolgt von reaktiven Veränderungen des subchondralen Knochens sowie Kapselveränderungen.

Unter dem Begriff "Spondylose" wird eine Arthrose der Wirbelkörper verstanden, die durch einen nicht entzündlichen Knorpelschwund der Wirbelkörper und Bandscheiben gekennzeichnet ist.

Die Applikation der erfindungsgemäßen Arzneimittel kann durch inhalative oder transdermale Applikation oder durch subkutane, intra-artikuläre, intraperitoneale oder intravenöse Injektion erfolgen. Bevorzugt ist die intra-artikuläre oder topische Applikation.

Geeignete feste oder galenische Zubereitungsformen sind beispielsweise Suspensionen, Emulsionen, oder injizierbare Lösungen sowie Präparate mit protrahierter Wirkstoff-Freigabe, bei deren Herstellung übliche Hilfsmittel Verwendung finden.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel 1 enthält. Bei Injektionslösungen in Ampullenform, kann diese Dosis bis zu etwa 300 mg, vorzugsweise aber etwa 10 bis 100 mg, bei Injektionslösungen für die intra-artikuläre Behandlung bis zu etwa 300 Mikrogramm, vorzugsweise 100 Mikrogramm, betragen.

Für die Behandlung eines erwachsenen Patienten sind je nach Wirksamkeit der Verbindung gemäß Formel I, Tagesdosen von etwa 0,01 mg/kg bis 10 mg/kg Wirkstoff bei systemischer Applikation indiziert, bei der Applikation von Injektionslösungen sind Tagesdosen von 0,001 mg/kg bis 0,005 mg/kg Wirkstoff indiziert und bei topischer oder inhalativer Applikation sind Tagesdosen von 0,01 mg/kg bis 5 mg/kg Wirkstoff indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Nachfolgend ist die Erfindung an Hand von Beispielen näher erläutert.

Die für die Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Oic: Octahydro-1 H-indol-2-carbonyl
- Thia: 2-Thienylalanyl.
- Tic: 1,2,3,4-Tetrahydroisochinolin-3-ylcarbonyl

HOE 140 wurde wie in EP 0 370 453 B1 beschrieben hergestellt.

### Pharmakologische Beispiele

Für die Analyse der krankheitsmodifizierenden Wirkung von HOE140 in einem Knorpelrelevanten Zellkulturmodel wurde die MMP3 Expression in der Chondrosarcom Zellline SW1353 (ATCC: HTB 94) analysiert. Für die Versuche wurden SW1353 Zellen unter Standard Bedingungen (37°C, 5% CO2) in DMEM-Glutamax mit 10% Fötalem Kälberserum (FKS) in Plastikkulturflaschen kultiviert. Nach ab-trypsinieren der Zellen wurden 50.000 Zellen je well einer 96-well Flachboden Platte in Medium ohne FKS ausgesät und mit der Verbindung HOE140 im Inkubator vorinkubiert. Nach einer Stunde erfolgte die Stimulation der Zellen durch Zugabe von humanem IL1-β (0,1 ng/ml, Roche) in einem Gesamtvolumen von 300 µl. Nach 24 Stunden Inkubation unter Standardbedingungen wurde der Zellkulturüberstand abgenommen, 5 Minuten zentrifugiert und bei -20 °C bis zur weiteren Analyse eingefroren. Die Analyse der MMP3 Expression in den Zellkulturüberständen erfolgt dann mittels eines kommerziellen MMP3 ELISA Testsystems (Amersham) nach Angaben des Herstellers. Parallel dazu wurde mit den verbleibenden Zellen ein WST-Zytotoxizitätstest durchgeführt. Dazu wurde das kommerzielle Testsystem der Firma Roche verwendet und die Messung nach Angaben des Herstellerprotokolls durchgeführt.

Die nachfolgende Tabelle 1 zeigt die Ergebnisse.
Bradykinin erhöht die MMP3 Freisetzung um mehr als 30%. Diese erhöhte Freisetzung von MMP 3 wurde mit HOE140 dosis-abhängig gehemmt.

**Tabelle 1 MMP-3 Freisetzung aus SW-Zellen**

| Stimulations-Parameter | MMP-3 Freisetzung | | Relativwerte bezogen auf Ausgangswert |
|---|---|---|---|
| | MW (OD450nm) | SD | |
| unstimuliert | 93 | 20 | |
| IL1α (0,05 ng/ml) | 328 | 17 | 100 |
| lL1α+Bradykinin (0,1 µM) | 433 | 32 | 132,0 |
| IL1α+Bradykinin (0,1 µM) +0,05 µM HOE140 | 458 | 50 | 139,6 |
| IL1α+ Bradykinin (0,1 µM) +0,1 µM HOE140 | 371 | 8 | 113,1 |
| IL1α+ Bradykinin (0,1 µM) +0,5 µM HOE140 | 309 | 18 | 94,2 |
| IL1α+ Bradykinin (0,1 µM) +1 µM HOE140 | 306 | 27 | 93,3 |

## Patentansprüche

1. Verwendung der Verbindung der Formel I
A-B X-E-F-K-(D)-TIC-GM-M-F'-I (I)
ausgewählt aus der Gruppe:
H-(D)-Arg-Arg-Pro-Hyp-Gly-Thia-Ser-(D)-Tic-Oic-Arg-OH,
H-(D)-Arg-Arg Pro-Pro-Gly Thia-Ser-(D)-Tic-Oic-Arg-OH,
H-(D)-Arg-Arg-Pro-Hyp-Gly-Phe-Ser-(D)-Tic-Oic-Arg-OH;
H-(D)-Arg-Arg-Hyp-Pro-G!y-Phe-Ser-(D)-Tic-Oic-Arg-OH und
H-(D)-Arg-Arg-Pro-Pro-Giy-Phe-Ser-(D)-Tic-Oic-Arg-OH
zur Herstellung von Arzneimitteln zur Prophylaxe und Therapie von Osteoarthrose, Spondylose oder Knorpelschwund nach Gelenktrauma oder längerer Gelenksruhigstellung nach Meniskus- oder Patellaverletzungen oder Bänderrissen.

2. Verwendung gemäß Anspruch 1, **dadurch gekennzeichnet, dass** die Verbindung D-Arginyl-L-arginyl-L-prolyl-L-protylglycyl-3-(2 thienyl)L-ralanyf-L-seryl-(3R)-1,2,3,4-tetrahydro-3-isochinolincarbonyl-(2S,3aS,7aS)-octahydro-1 H-indol-2-carbonyf-L-arginin eingesetzt wird.

3. Verwendung der Verbindung der Formel I gemäß der Ansprüche 1 oder 2, **dadurch gekennzeichnet, dass** die Applikation durch subkutane, intra-artikuläre, intraperitoneale oder intravenöse Injektion oder transdermale Applikation erfolgt.

## Claims

1. The use of the compound of the formula I
A-B-X-E-F-K-(D)-TIC-GM-M-F'-I (I)
selected from the group:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH and
H-(D)-Arg-Arg-Pro-Pro-Gly-Phe-Ser-(D)-Tic-Oic-Arg-OH
for the production of pharmaceuticals for the prophylaxis and therapy of osteoarthrosis, spondylosis, chondroporosis after joint trauma or relatively long immobilization of a joint after meniscus or patella injuries or torn ligaments.

2. The use as claimed in claim 1, wherein the compound D-arginyl-L-arginyl-L-prolyl-L-prolylglycyl-3-(2-thienyl)-L-alanyl-L-seryl-(3R)-1,2,3,4-tetrahydro-3-isoquinolinecarbonyl-(2S,3aS,7aS)-octahydro-1H-indole-2-carbonyl-L-arginine is employed.

3. The use of the compound of the formula I as claimed in claims 1 or 2, wherein the administration is carried out by subcutaneous, intraarticular, intraperitoneal or intravenous injection or transdermal administration.

## Revendications

1. Utilisation du composé de formule I
A-B-X-E-F-K-(D)-TIC-GM-M-F'-I (I)
choisi dans le groupe :
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH;
H-(D)-Arg-Arg-Hyp-Pro-Gly-Phe-Ser-(D)-Tic-Oic-Arg-OH et
H-(D)-Arg-Arg-Pro-Pro-Gly-Phe-Ser-(D)-Tic-Oic-Arg-OH
pour la préparation de médicaments destinés à la prophylaxie et la thérapie de l'ostéoarthrose, de la spondylose ou de l'atrophie du cartilage après un traumatisme articulaire ou un repos prolongé des articulations après des blessures au ménisque ou à la rotule ou des déchirures de ligaments.

2. Utilisation selon la revendication 1, **caractérisée en ce qu'**on utilise le composé D-arginyl-L-arginyl-L-prolyl-L-prolylglycyl-3-(2-thiényl)-L-alanyl-L-séryl-(3R)-1,2,3,4-tétrahydro-3-isoquinoléinecarbonyl-(25,3aS,7aS)-octahydro-1H-indole-2-carbonyl-L-arginine.

3. Utilisation du composé de formule I selon les revendications 1 ou 2, **caractérisée en ce que** l'administration est réalisée par une injection sous-cutanée, intra-articulaire, intrapéritonéale ou intraveineuse ou par une administration transdermique.
